# FASCICULE DE BREVET EUROPEEN

(11) **EP 3 416 944 B1**
(45) Date de publication et mention de la délivrance du brevet: **04.12.2019**
(21) Numéro de dépôt: 17705855.9
(22) Date de dépôt: 16.02.2017
(51) Int. Cl.: C07C 319/28, C07C 327/22

(54) **PROCÉDÉ DE PRÉPARATION DE COMPOSÉS AMINOTHIOLESTER ET LEURS SELS**
VERFAHREN ZUR HERSTELLUNG VON AMINOTHIOLESTERVERBINDUNGEN UND SALZEN DAVON
PROCESS FOR PREPARING AMINOTHIOL ESTER COMPOUNDS AND SALTS THEREOF

(30) Priorité: 17.02.2016 FR 1651283
(43) Date de publication de la demande: 26.12.2018
(73) Titulaire: Advanced Biodesign, 69800 Saint Priest (FR)
(72) Inventeur: ROOL, Patrice, 63200 Riom (FR); DE CARNE-CARNAVALET, Benoit, 63200 Riom (FR)
(74) Mandataire: Lavoix
(86) Numéro de dépôt international: PCT/EP2017/053457
(87) Numéro de publication internationale: WO 2017/140754

(56) Documents cités:
- US-A1- 2003 181 443
- QUASH G ET AL: "Aldehyde dehydrogenase inhibitors: @a,@b-Acetylenic N-substituted aminothiolesters are reversible growth inhibitors of normal epithelial but irreversible apoptogens for cancer epithelial cells from human prostate in culture", EUROPEAN JOURNAL OF MEDICINAL CHEMISTRY, EDITIONS SCIENTIFIQUE ELSEVIER, PARIS, FR, vol. 43, no. 5, 1 mai 2008 (2008-05-01), pages 906-916, XP022639683, ISSN: 0223-5234, DOI: 10.1016/J.EJMECH.2007.06.004 [extrait le 2008-05-01]
- NASEEM SADIA ET AL: "Photo-isomerization upshifts the pKaof the Photoactive Yellow Protein chromophore to contribute to photocycle propagation", JOURNAL OF PHOTOCHEMISTRY AND PHOTOBIOLOGY, A: CHEMISTRY, vol. 270, 2013, pages 43-52, XP028704667, ISSN: 1010-6030, DOI: 10.1016/J.JPHOTOCHEM.2013.06.019
- BENJAMIN KOEPPE ET AL: "Solvent and H/D Isotope Effects on the Proton Transfer Pathways in Heteroconjugated Hydrogen-Bonded Phenol-Carboxylic Acid Anions Observed by Combined UV-vis and NMR Spectroscopy", JOURNAL OF THE AMERICAN CHEMICAL SOCIETY, vol. 135, no. 20, 22 mai 2013 (2013-05-22) , pages 7553-7566, XP055307350, US ISSN: 0002-7863, DOI: 10.1021/ja400611x -& Benjamin Koeppe ET AL: "Solvent and H/D isotope effects on the proton transfer pathways in heteroconjugated hydrogen-bonded phenol-carboxylic acid anions observed by combined UV-vis and NMR spectroscopy", Journal of the American Chemical Society, 22 mai 2013 (2013-05-22), page 7553, XP055307400, United States Extrait de l'Internet: URL:http://pubs.acs.org/doi/suppl/10.1021/ ja400611x

## Description

La présente invention concerne un procédé de préparation de composés aminothiolester et des sels desdits composés.

Un procédé de préparation de tels produits est notamment décrit dans US2003/0181443.

Cependant, la première étape de ce procédé met en œuvre le 3-chloro-3 methylbut-1-yne pour lequel l'approvisionnement peut être délicat. De plus, la pureté du produit commercial est variable ce qui peut avoir un impact sur le reste de la chaîne réactionnelle, notamment en termes de rendement. Par ailleurs, les étapes de purification pour cette première étape semblent difficilement transposables à grande échelle et le produit de cette première étape a tendance à se sublimer ce qui rend d'autant plus délicates ces étapes de purification.

La seconde étape de ce procédé met en œuvre de l'oxysulfure de carbone (COS) qui est un produit toxique et dont l'approvisionnement est aléatoire suivant les pays.

Il convient donc de fournir un procédé permettant de résoudre les inconvénients de l'art antérieur.

Un objectif de la présente invention est de fournir un procédé de préparation de composés aminothiolester et de leurs sels limitant la mise en œuvre de produits toxiques, notamment ne mettant pas en œuvre de COS.

Un autre objectif de l'invention est de fournir un procédé mettant en œuvre des réactifs ne présentant pas de problème d'approvisionnement, et notamment un procédé ne mettant pas en œuvre le 3-chloro-3-methylbut-1-yne commercial.

Un autre objectif encore de l'invention est de fournir un procédé pour lequel les produits et réactifs ne risquent pas de sublimer lors des différentes étapes du procédé et notamment lors de la purification.

D'autres objectifs apparaitront à la lecture de la description qui suit.

Ces objectifs sont remplis par la présente invention qui propose un procédé de préparation de composés de formule (I) 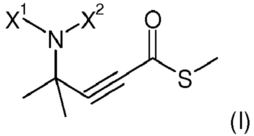 dans laquelle X¹ et X², identiques ou différents, sont choisis parmi les alkyles en C₁ à C₇, le phényle, le benzyle ou X¹ et X² forment avec l'atome d'azote qui les porte, un heterocycle, en particulier piperidine ou morpholine, le procédé comprenant les étapes suivantes :
a) réaction d'un composé de formule (II) avec un acide inorganique ou un acide organique 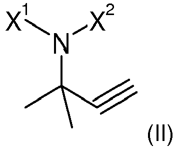
b) réaction du composé obtenu à l'étape a) avec une base présentant un pKa supérieur à 25;
c) réaction du composé obtenu à l'étape b) avec du CO₂ ;
d) réaction du composé obtenu à l'étape c) avec un chloroformiate d'alkyle, un réactif susceptible de former, avec le composé obtenu à l'étape c), un halogénure d'acide ou un réactif susceptible de former, avec le composé obtenu à l'étape c), un anhydride mixte ;
e) réaction du composé obtenu à l'étape d) avec un composé précurseur d'anion SMe⁻.

Les étapes du procédé peuvent être réalisées sans purification et sans isoler les produits intermédiaires. Comme précisé par la suite, des purifications intermédiaires peuvent être mises en œuvre, notamment pour améliorer le rendement de l'étape suivante.

Dans le cadre de la présente invention, par groupe C₁-C₇ alkyle, on entend un groupe aliphatique hydrocarboné, linéaire ou ramifié, comprenant, sauf mention contraire, de 1 à 7 atomes de carbone. De préférence sont visés les groupes alkyles comprenant de 1 à 3 atomes de carbone. Par « ramifié » on entend que un ou plusieurs groupes alkyle, par exemple méthyle, éthyle ou propyle sont attachés à la chaîne alkyle linéaire. Des exemples de groupe alkyle incluent méthyle, éthyle, n-propyle, i-propyle, n-butyle, t-butyle, 2,2-diméthylbutyl, n-pentyle, n-hexyle, n-heptyle, en particulier méthyle.

En particulier, les composés de formule (I) sont des composés pour lesquels X¹ et X², identiques ou différents, sont choisis parmi méthyle, phenyle, benzyle, au moins un de X¹ ou X² étant un méthyle, ou X¹ et X² forment avec l'atome d'azote qui les porte un cycle piperidine ou morpholine.

De préférence, les composés de formule (I) sont choisis parmi :
- S-methyl 4-methyl-4-(piperidin-1-yl)pent-2-ynethioate;
- S-methyl 4-[benzyl(methyl)amino]-4-methylpent-2-ynethioate;
- S-methyl 4-methyl-4-[methyl(phenyl)amino]pent-2-ynethioate;
- S-methyl 4-methyl-4-(morpholin-4-yl)pent-2-ynethioate;
- S-methyl 4-(dimethylamino)-4-methylpent-2-ynethioate;
- S-methyl 4-(diheptylamino)-4-methyl-pent-2-ynethioate; et/ou
- S-methyl 4-[heptyl(methyl)amino]-4-methyl-pent-2-ynethioate.

De préférence, le composé de formule (I) est le S-methyl 4-(dimethylamino)-4-methylpent-2-ynethioate.

Le composé de formule (II) selon l'invention, éventuellement sous forme salifiée, peut être obtenu par toute méthode connue de l'homme du métier.Dans un mode de réalisation particulier, le composé de formule (II) peut être obtenu par réaction du 3-chloro-3-méthylbut-1-yne avec un composé de formule X¹X²NH. De telles réactions sont notamment décrites dans Hennion et al, JACS, 1960, 4908-4912. De préférence dans ce mode de réalisation, le composé de formule (II) est obtenu par une étape a1) de réaction du 3-chloro-3-méthylbut-1-yne avec X¹X²NH dans un milieu aqueux. De préférence, cette étape a1) est mise en œuvre avec 1 à 3 équivalents de X¹X²NH par rapport au nombre de moles de 3-chloro-3-méthylbut-1-yne. De préférence, l'étape a1) est mise en œuvre à une température comprise entre 0 et 50°C, de préférence entre 10 et 40°C, de préférence à température ambiante, c'est-à-dire à une température comprise entre 20 et 25°C. Le composé obtenu à l'étape a1) peut être purifié avant sa mise en œuvre dans l'étape a). La purification peut notamment constituer en une ou plusieurs filtrations, par exemple en 1 filtration ou en une succession de 2 à 10 filtrations, de préférence en une succession de 2 à 5 filtrations, par exemple en 4 filtrations. Dans ce mode de réalisation, le 3-chloro-3-méthylbut-1-yne peut être commercial ou peut être obtenu par une étape a0) de réaction du 2-méthylbut-3-yn-2-ol avec un acide minéral, de préférence l'acide chlorhydrique, en présence d'un catalyseur au cuivre. De préférence, dans l'étape a0), l'acide est mis en œuvre en une quantité de 3 à 10, de préférence 5, équivalents par rapport au nombre de moles de 2-méthylbut-3-yn-2-ol. De préférence, l'étape a0) est réalisée en présence de chlorure de calcium (par exemple CaCl₂), de chlorure de cuivre (par exemple CuCl₂ ou CuCl, de préférence CuCl₂) et de Cu. De préférence, dans l'étape a0), le chlorure de calcium est mis en œuvre dans une proportion de 0,1 à 1, de préférence de 0,2 à 0,7, équivalent par rapport au nombre de moles de 2-méthylbut-3-yn-2-ol. De préférence, dans l'étape a0), le chlorure de cuivre est mis en œuvre dans une proportion de 0,1 à 1, de préférence de 0,2 à 0,7, équivalent par rapport au nombre de moles de 2-méthylbut-3-yn-2-ol. De préférence, dans l'étape a0), le Cu est mis en œuvre en quantité catalytique, notamment dans une proportion de 0,005 à 0,1, de préférence de 0,007 à 0,05, équivalent par rapport au nombre de moles de 2-méthylbut-3-yn-2-ol. De préférence, l'étape a0) est mise en œuvre à une température comprise entre -78°C et 10°C, de préférence entre -50°C et 0°C.

Dans un autre mode de réalisation de l'invention, le composé de formule (II) peut être obtenu par réaction entre un acétate ou un phosphate du 2-méthyl-3-butyn-2-ol et un composé de formule NHX¹X² en présence d'un catalyseur, notamment au cuivre. Une telle réaction est notamment décrite dans J.Org.Chem. 1994, 59, 2282-2284. Dans ce mode de réalisation, le composé de départ est un composé de formule H-C≡C(Me)₂-OR avec R qui représente OP(O)(OEt)₂ ou OAc. De préférence, la réaction est mise en œuvre en présence de chlorure de cuivre, notamment CuCl.

Dans un autre mode de réalisation, le composé de formule (II) peut être obtenu par diméthylation de la 1,1-diméthylpropargylamine (commerciale), notamment par l'acide formique en présence de paraformaldéhyde aqueux, tel que décrit dans J.Org.Chem, 1957, 22, 840-843.

L'étape a) est, de préférence, mise en œuvre en présence d'un solvant, ledit solvant étant choisi parmi les solvants qui solubilisent l'acide inorganique ou l'acide organique mais qui ne solubilisent pas le produit formé lors de l'étape a). Par exemple, le solvant est choisi parmi les alcools, notamment méthanol, isopropanol, le dioxane et l'éther. De préférence, le solvant est le dioxane.

De préférence, dans l'étape a), l'acide inorganique ou organique est introduit en solution dans ledit solvant, de préférence, la solution comprend de 1 à 3 équivalents en moles d'acide.

De préférence, dans l'étape a), l'acide inorganique est choisi parmi l'acide chlorhydrique, l'acide phosphorique, l'acide nitrique, l'acide sulfurique, de préférence l'acide chlorhydrique.

De préférence, dans l'étape a), l'acide organique est choisi parmi les acides carboxyliques, les acide sulfoniques.

De préférence, dans l'étape a), l'acide est un acide inorganique et est de préférence l'acide chlorhydrique.

De préférence, l'étape a) est mise en œuvre à une température comprise entre 0 et 50°C, de préférence entre 0 et 20°C.

Le sel obtenu à l'étape a) peut être purifié, notamment par filtration puis recristallisation, notamment dans les conditions définies par Hennion et al., J. Am. Chem. Soc. 1957, 79, 2142-2145. De préférence, le sel est purifié par recristallisation, de préférence dans un mélange d'ester et d'alcool, notamment dans un mélange d'acétate d'éthyle et d'éthanol.

De façon avantageuse, l'étape a) permet l'obtention d'un produit sous forme de sel qui ne sublime pas, ce qui permet de surmonter les inconvénients de l'art antérieur.

La base mise en œuvre à l'étape b) peut être choisie parmi les bases connues de l'homme du métier pour déprotoner un alcyne vrai. La base est choisie parmi les bases présentant un pKa supérieur à 25. De préférence, la base mise en œuvre à l'étape b) est choisie parmi les bases à base de lithium ou de magnésium, de préférence la base est choisie parmi le butyllithium, ou l'hexyllithium. De préférence la base est le butyllithium et plus particulièrement le *n-* butyllithium.

De préférence, la quantité de base mise en œuvre est comprise entre 1,5 et 4, de préférence entre 1,5 et 2,5, équivalents par rapport au nombre de moles du sel obtenu à l'étape a). De préférence lorsque le composé de formule (II) selon l'invention est utilisé en tant que tel, la quantité de base mise en œuvre est de 1,5 équivalents et lorsque le composé de de formule (II) selon l'invention est utilisé sous forme de sel, la quantité de base mise en œuvre est de 2,5 équivalents.

L'ajout de la base est une réaction exothermique. Il est donc préférable d'ajouter la base à une température inférieure à 0°C, de préférence à une température comprise entre -78 et 0°C, de préférence entre -78 et -50°C, après l'ajout la température peut ne plus être contrôlée et le milieu réactionnel est de préférence laissé remonter à 0°C.

L'étape b) peut être mise en œuvre en présence d'un solvant, notamment choisi parmi les éthers, notamment choisi parmi le tétrahydrofurane, le méthyltetrahydrofurane, le diéthyléther, le dioxane, le diisopropyléther, le méthyltertiobutyléther. De préférence, le solvant est le tétrahydrofurane (THF).

De préférence, l'ajout de CO₂ à l'étape c) peut se faire par barbotage de CO₂ dans le milieu réactionnel ou par production de CO₂ gazeux in situ par ajout dans le milieu réactionnel de carboglace (ou glace carbonique). Le CO₂ est de préférence mis en œuvre en large excès, notamment, dans le cas où la carboglace est mise en œuvre pour la production in-situ de CO₂ gazeux, la quantité de carboglace ajoutée est comprise entre 1 et 50 fois le poids du composé aminé obtenu à l'étape b).

L'ajout de CO₂ à l'étape c) (via barbotage ou carboglace) s'accompagne d'une augmentation de température. Ainsi, et de préférence, le CO₂ est ajouté à une température inférieure à 0°C, de préférence à une température comprise entre -78 et 0°C, de préférence entre -78 et -50°C, après l'ajout la température peut ne plus être contrôlée et le milieu réactionnel est de préférence laissé remonter à 0°C.

De façon avantageuse, le produit obtenu à l'étape c) peut être purifié par toute méthode connue de l'homme du métier. L'étape de purification peut notamment consister en un ajout de méthanol pour piéger la base, en un ajout d'acide inorganique, notamment HCl, pour isoler le produit sous forme de sel ou en un ajout d'eau ou méthanol pour piéger la base, puis l'ajout d'un solvant permettant la précipitation du produit formé, le solvant étant notamment choisi parmi acétonitrile, ou le méthanol, de préférence acétonitrile, et la filtration du milieu réactionnel obtenu. Dans cette étape de purification, le produit de l'étape c) est dans le retenta de la filtration. Un autre procédé de purification est de former, après quenchage de la base par ajout de méthanol, un chlorhydrate. Ces méthodes sont bien connues de l'homme du métier.

L'objectif de l'étape d) est d'activer la fonction acide du composé obtenu à l'étape c).

De préférence, le chloroformiate d'alkyle mis en œuvre à l'étape d) présente un alkyle en 1 à 6 atomes de carbone, pouvant comprendre au moins une double liaison. De préférence, c'est un chloroformiate de méthyle, d'éthyle, d'isoprényl, de tertiobutyle ou d'isobutyle, de préférence le chloroformiate d'isobutyle.

L'Homme du métier est à même de déterminer les réactifs susceptibles de former avec le composé obtenu à l'étape c) un anhydride mixte ou un halogénure d'acide.

De préférence, les réactifs susceptibles de former avec le composé obtenu à l'étape c) un anhydride mixte sont choisis parmi les chlorures d'acide, par exemple le chlorure de pivaloyle.

De préférence, les réactifs susceptibles de former avec le composé obtenu à l'étape c) un halogénure d'acide sont choisis parmi les réactifs susceptibles de former avec le composé obtenu à l'étape c) un chlorure ou bromure d'acide, par exemple ils sont choisis parmi SOCl₂, COCl₂, PCl₃, PCl₅, PBr₃ ou PPh₃Br₂.

La quantité de chloroformiate d'alkyle, de réactif susceptible de former avec le composé obtenu à l'étape a) un anhydride mixte ou un halogénure d'acide, mise en œuvre à l'étape d) peut être comprise entre 1 et 3, de préférence entre 1 et 2, équivalents par rapport au nombre de moles du composé obtenu à l'étape c).

De préférence l'étape d) est mise en œuvre avec un chloroformiate d'alkyle.

De préférence, l'étape d) est mise en œuvre à une température comprise entre -78°C et 50°C, de préférence entre -0 et 25 °C.

Si le produit de l'étape c) n'est pas purifié alors aucun solvant supplémentaire n'est ajouté lors de l'étape d). Dans le cas contraire, l'étape d) est de préférence mise en œuvre en présence d'un solvant notamment choisi parmi ceux décrits pour l'étape b), de préférence le THF.

De préférence, dans le cadre de la présente invention, les composés précurseurs d'anion SMe⁻, sont des composés susceptibles, notamment dans le milieu réactionnel ou en présence d'une base de générer l'anion SMe⁻. Ces composés sont notamment choisis parmi les sels de formule XSMe dans laquelle X représente un métal alcalin ou alcalino-terreux, par exemple Na, le methyl mercaptan, ou (SMe)₂. Lorsque le méthyl mercaptan ou (SMe)₂ sont mis en œuvre, il est nécessaire d'ajouter une base dans le milieu réactionnel, notamment une base dont le pKa est supérieur à 14, par exemple sodium, alcoolate (ex alcoolate de sodium, par exemple éthanolate de sodium), éthylate, méthylate , tertiobutylate de sodium,.... De préférence, dans l'étape e), le composé précurseur d'anion SMe⁻ est choisi parmi les composés de formule XSMe dans laquelle X représente un métal alcalin ou alcalino-terreux, de préférence NaSMe. Dans l'étape e), le NaSMe peut être mis en œuvre sous forme de poudre ou sous forme de solution dans l'eau. Le composé précurseur d'anion SMe⁻ est mis en œuvre, notamment de façon à obtenir de 1 à 3, de préférence de 1 à 2 équivalents d'anions SMe⁻ par rapport au nombre de moles du composé obtenu à l'étape d).

De préférence, l'étape e) est mise en œuvre à une température comprise entre 0 et 50°C, de préférence à température ambiante (comprise entre 20 et 25°C).

De préférence, le procédé de l'invention comprend les étapes suivantes :
a0) optionnellement une étape a0) de réaction du 2-méthylbut-3-yn-2-ol avec un acide inorganique, notamment acide chlorhydrique en présence d'un catalyseur au cuivre ;
a1) optionellement, dans le cas où l'étape a0) a été mise en œuvre, réaction du 3-chloro-3-méthylbut-1-yne, le cas échéant obtenu à l'étape a0) avec X¹X²NH dans un milieu aqueux ;
a) réaction d'un composé de formule (II) obtenu à l'étape a1) le cas échéant ou commercial avec un acide inorganique, de préférence HCl 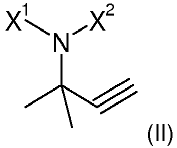
b) réaction du composé obtenu à l'étape a) avec une base, de préférence alkyllithium, de préférence butyllithium et plus particulièrement le *n-* butyllithium;
c) réaction du composé obtenu à l'étape b) avec du CO₂ ;
d) réaction du composé obtenu à l'étape c) avec un chloroformiate d'alkyle, de préférence chloroformiate d'isobutyle;
e) réaction du composé obtenu à l'étape d) avec SMeNa.
Les caractéristiques particulières et préférées des différentes étapes décrites ci-dessus, notamment température, quantité des réactifs,... s'appliquent également à la description de ce procédé préféré.

La présente invention concerne également la préparation d'un sel d'un composé de formule (I) comprenant les étapes de :
i) préparation du composé de formule (I) selon le procédé de l'invention ;
ii) réaction du composé obtenu à l'étape i) avec l'acide correspondant au sel souhaité.

De préférence, l'étape ii) est mise en œuvre en présence d'alcool ou d'un mélange d'alcool et d'éther. De préférence l'alcool est choisi parmi éthanol, méthanol, isopropanol De préférence, l'éther est choisi parmi le tétrahydrofurane, le méthyltétrahydrofurane, le diéthyléther, le dioxane, le diisopropyléther, le méthyltertiobutyléther, l'acétate d'éthyle. De préférence, le solvant est le tétrahydrofurane (THF). De préférence, l'éther correspondant à l'alcool mis en œuvre. De préférence, l'alcool est l'éthanol et l'éther est le diéthyléther.

Le composé obtenu est de préférence filtré et peut être purifié par recristallisation, par exemple dans un alcool notamment isopropanol.

De préférence, le composé de formule (I) est le DIMATE, composé pour lequel X¹ et X² représentent méthyle. Le DIMATE ou S-methyl 4-(dimethylamino)-4-methylpent-2-ynethioate (numéro CAS 350229-29-7, poids moléculaire: 185.29 g.mol⁻¹, formule: C₉H₁₅NOS), est un composé de formule (IA):

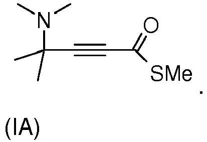

De préférence, le sel du composé de formule (I) est le fumarate de DIMATE, l'acide mis en œuvre à l'étape ii) étant alors l'acide fumarique.

De préférence, l'étape a0) correspond au schéma réactionnel suivant :

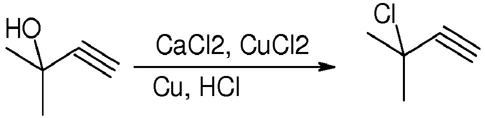

De préférence, l'étape a1) correspond au schéma réactionnel suivant :

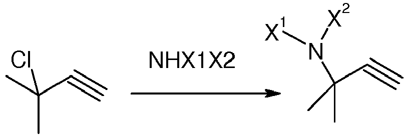

De préférence, l'étape a) correspond au schéma réactionnel suivant :

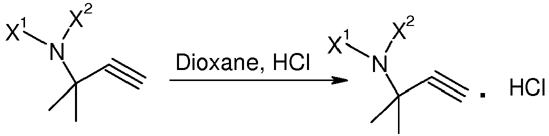

De préférence, l'étape b) correspond au schéma réactionnel suivant :

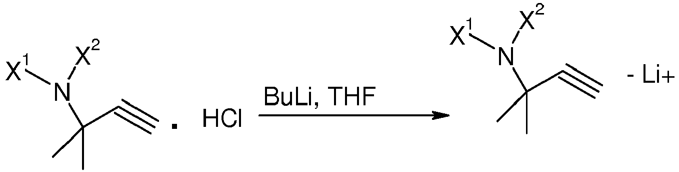

De préférence, l'étape c) correspond au schéma réactionnel suivant :

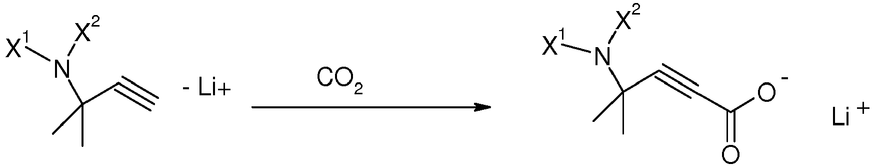

De préférence, l'étape d) correspond au schéma réactionnel suivant :

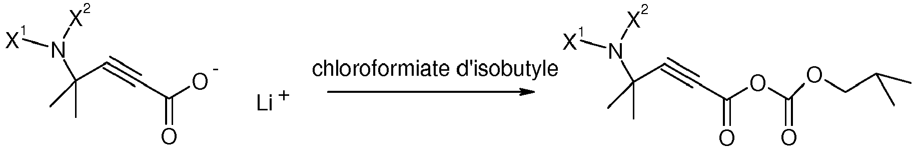

De préférence, l'étape e) correspond au schéma réactionnel suivant :

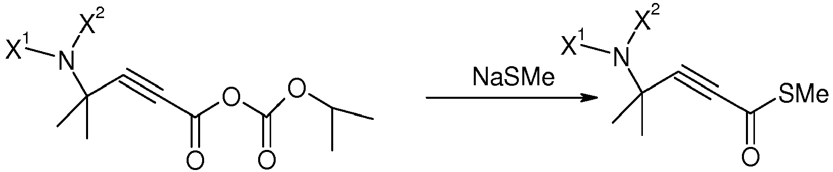

De préférence, l'étape ii) correspond au schéma réactionnel suivant :

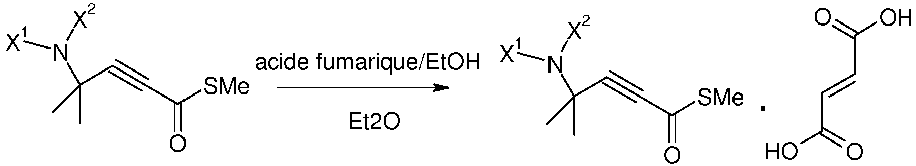

La présente invention va maintenant être décrite à l'aide d'exemples non limitatifs.

### Exemple 1 : Préparation du S-methyl 4-(dimethylamino)-4-methylpent-2-ynethioate (Dimate) et du fumarate de celui-ci

### 1. Etape a0)

Dans un ballon sont ajoutés 226,4 g de CaCl₂ (2,040 mol), 219,70 g de CuCl₂ (1,634 mol) et 2,440 g de Cu (0,0384 mol). Le mélange est placé à -20°C. 1770 ml d'HCl (57,8 mol) sont ajoutés en 30 minutes. Le mélange réactionnel est agité pendant 1 heure à -20°C. 300 g de 2-méthyl-3-butyn-2-ol (4 mol) sont ajoutés en 45 minutes à -20°C. Après 2h30 à -20°C et retour à la température ambiante (20-25°C), le mélange est laissé à décanter, puis les phases sont séparées et les phases organiques sont lavées par 6 fois avec 140 ml d'HCI 37% puis avec de l'eau. Les phases organiques sont ensuite regroupées et séchées sur K₂CO₃ puis distillées, sur K₂CO₃ à 40-45°, 350 mbar. 268 g de 3-chloro-3 methylbut-1-yne sont obtenus (rendement 60%).

### 2. Etape a1)

268 g du composé obtenu à l'étape a0) (2,61 mol) sont ajoutés dans un ballon avec 476 ml de diméthylamine (40% en poids en solution aqueuse) (9,41 mol). Au bout de 16h de réaction à température ambiante, le milieu réactionnel est filtré, le filtrat obtenu est mélangé puis filtré, cette étape est répétée 2 fois encore. 156,5 g de l'amine sont obtenues (rendement 53,8%).

### 3. Etape a)

156,5 g de l'amine obtenue à l'étape a1) sont dissous dans 7 volumes de dioxane et 3 volumes de dioxane.HCl 6,5M. A la fin de la réaction à température ambiante, le sel est filtré et lavé avec du dioxane puis recristallisé dans un mélange EtOH/AcOEt (70/30 8 volumes). 146 g du sel de l'amine sont obtenus (rendement de 37,8%).

### 4. Etapes b), c), d) et e)

500 mg du sel obtenu à l'étape a) (0,003386 mol) est introduit, sous atmosphère d'azote, dans un ballon avec 5 ml de tetrahydrofuranne (0,06 mol). Le mélange réactionnel est refroidi à -78°C. 3 ml d'une solution 2,5M de n-BuLi dans l'hexane sont ajoutés. Le mélange réactionnel est laissé jusqu'à atteindre 0°C. 1 g de CO₂ (0,03 mol) est ajouté (par bullage) à 0°C. Le mélange réactionnel est laissé jusqu'à atteindre la température ambiante (20-25°C). Le mélange réactionnel est ensuite refroidi à 0°C et 0,486 ml de chloroformiate d'isobutyle (0,00372 mol) sont ajoutés. Le mélange réactionnel est laissé jusqu'à atteindre la température ambiante (20-25°C). 0,285 g de NaSMe (0,00406 mol) sont ajoutés. Après 20 min à température ambiante (20-25°C), de l'eau et de l'acétate d'éthyle sont ajoutés. Les phases aqueuses sont extraites avec de l'acétate d'éthyle et les phases organiques sont lavées avec une solution saturée de NaCl, séchées sur MgSO4 et concentrées pour donner une huile jaune (600 mg). Le produit obtenu est purifié sur gel de silice avec un éluant cyclohexane/AcOEt 9/1 à 7/3 et le DIMATE est obtenu (157 mg) sous forme d'huile incolore (rendement 25,02%).

### 5. Etape ii)

11,4 g d'acide fumarique (0,09821 mol) sont dissous dans 450 ml d'éthanol (7,7 mol) à température ambiante, une sonication pouvant permettre d'atteindre la solubilisation totale. Cette solution est ajoutée au goutte à goutte sur une solution de 19,2 g de DIMATE (0,1036 mol) dans 250 ml d'éther (2,4 mol). Après 30 minutes, le mélange obtenu est filtré, lavé avec de l'éther (50 ml), séché sous vide et le fumarate de DIMATE est obtenu sous la forme d'un solide blanc (19,1 g, rendement 64,5%).

### Composé obtenu à la fin de l'étape e) (DIMATE)

- **¹H NMR** (300MHz, CDCl₃) : δ = 1.42 (s, 6H, (CH₃)₂), 2.31 (s, 6H, N(CH₃)₂), 2.39 ppm (s, 3H, CH₃S).
- **¹H NMR** (300MHz, DMSO-*d₆*) : δ = 1.35 (s, 6H, (CH₃)₂), 2.20 (s, 6H, N(CH₃)₂), 2.39 ppm (s, 3H, CH₃S).
- **¹³C NMR** (75MHz, CDCl₃) : δ = 12.1 (CH₃S),, 27.5 ((CH₃)₂), 40.0 (N(CH₃)₂), 54.7 (C), 81.5 (C), 94.7 (C), 175.8 (COS) ppm

### Composé obtenu à la fin de l'étape ii) (DIMATE FUMARATE):

- **¹H NMR** (300MHz, DMSO-*d₆*) : δ = 1.36 (s, 6H, (CH₃)₂), 2.21 (s, 6H, N(CH₃)₂), 2.39 (s, 3H, CH₃S), 6.63 (s, 2H, =CH), 13.12 ppm (s, 2H, CO₂H).
- **¹H NMR** (300MHz, D₂O) : δ = 1.79 (s, 6H, (CH₃)₂), 2.46 (s, 3H, CH₃S), 2.98 (s, 6H, N(CH₃)₂), 6.68 ppm (s, 2H, =CH).
- **¹³C NMR** (75MHz, DMSO-*d₆*) : δ = 12.3 (CH₃S), 27.2 ((CH₃)₂), 39.8 (N(CH₃)₂), 54.7 (C), 81.2 (C), 95.4 (C), 134.1 (=CH), 166.1 (CO2H), 175.8 (COS) ppm

### Exemple 2 : Préparation du S-methyl 4-[heptyl(methyl)amino]-4-methyl-pent-2-ynethioate

Le procédé détaillé dans les étapes a0) à e) est reproduit en utilisant l'amine suivante (composé de formule II): N-(1,1-dimethylprop-2-ynyl)-N-methyl-heptan-1-amine. A la N-methylheptan-1-amine (3.84 g, 29.7 mmol) en solution dans le THF (50 mL) sont ajoutés successivement à température ambiante (20-25°C) la diisopropylamine (4.3 mL, 24.7 mmol), Cu (0.150 g, 2.4 mmol), CuCl (0.150 g, 1.5 mmol) puis le 3-chloro-3 méthylbut-1-yne (2.54 g, 24.8 mmol)(obtenu tel que mentionné dans l'exemple 1). Le milieu réactionnel est agité pendant 16h. Après ajout de 25 mL d'eau et décantation, la phase aqueuse est extraite à l'acétate d'éthyle (3x30 mL). Les phases organiques réunies sont lavées successivement par NH₄OH 10% aqueux (25 mL) et une solution aqueuse saturée de NaCl (25 mL). Après séchage sur Na₂SO₄, concentration à l'évaporateur rotatif, le résidu est purifié par distillation sous vide dans un appareil de Kugelrohr (2399,8 Pascal (18 Torr), température du four: 100 à 150°C). 2.10 g de N-(1,1-dimethylprop-2-ynyl)-N-methyl-heptan-1-amine sont obtenus sous forme d'huile incolore, rdt 43%.
- Poids moléculaire =195,35
- Formule chimique=C13H25N.
- **¹H NMR** (300 MHz, DMSO) δ 3.08 (s, 1H), 2.37 - 2.28 (m, 2H), 2.13 (s, 3H), 1.39 - 1.18 (m, 10H), 1.27 (s, 6H), 0.91 - 0.80 (m, 3H).
- **¹³C NMR** (75 MHz, CDCl₃) δ 86.29 (C), 70.72 (CH), 54.59 (C), 52.36 (CH₂), 36.27 (CH₃), 31.95 (CH₂), 29.36 (CH₂), 29.05 (CH₂), 28.49 (CH₃), 27.61 (CH₂), 22.69 (CH₂), 14.14 (CH₃).
- **ESI-LRMS** 196.0 [M+H]⁺.

Le produit final est obtenu à partir de 0.51 g (2.6 mmol) de N-(1,1-diméthylprop-2-ynyl)-N-méthyl-heptan-1-amine avec un rendement de 25% sous forme de liquide (base libre) (0.176 g)

### Composé obtenu à la fin de l'étape e) (S-methyl 4-[heptyl(methyl)amino]-4-methyl-pent-2-ynethioate)

- **¹H NMR** (300 MHz, DMSO) δ 2.38 (s, 3H), 2.37 (t, *J* = 7.0 Hz, 2H), 2.18 (s, 3H), 1.35 (s, 6H), 1.42 - 1.20 (m, 10H), 0.86 (t, *J* = 6.7 Hz, 3H).
- **¹³C NMR** (75 MHz, CDCl₃) δ 176.38 (C), 96.50 (C), 81.03 (C), 54.98 (C), 52.39 (CH₂), 36.39, 31.85 (CH₂), 29.25 (CH₂), 28.79 (CH₂), 27.81 (2CH₃), 27.41 (CH₂), 22.62 (CH₂), 14.09 (CH₃), 12.41 (CH₃).
- **ESI-LRMS** 270.2 [M+H]⁺.

### Exemple 3 : Préparation du S-methyl 4-(diheptylamino)-4-methyl-pent-2-ynethioate

Le procédé détaillé dans les étapes a0) à e) est reproduit en utilisant l'amine suivante (composé de formule II) : N-(1,1-dimethylprop-2-ynyl)-N-heptyl-heptan-1-amine.

A la N-heptylheptan-1-amine (5.2 g, 24.4 mmol) en solution dans le THF (41 mL) sont ajoutés successivement à température ambiante (20-25°C) la diisopropylamine (3.54 mL, 20.3 mmol), Cu (0.120 g, 1.9 mmol), CuCl (0.120 g, 1.2 mmol) puis le 3-chloro-3 methylbut-1-yne (2.08 g, 20.3 mmol) )(obtenu tel que mentionné dans l'exemple 1). Le milieu réactionnel est agité pendant 16h. Après ajout de 25 mL d'eau et décantation, la phase aqueuse est extraite à l'acétate d'éthyle (3x30 mL). Les phases organiques réunies sont lavées successivement par NH₄OH 10% aqueux (25 mL) et une solution aqueuse saturée de NaCl (25 mL). Après séchage sur Na₂SO₄, concentration à l'évaporateur rotatif, le résidu est purifié par distillation sous vide dans un appareil de Kugelrohr (2399,8 Pascal (18 Torr), température du four: 100 à 150°C). 1.50 g de N-(1,1-dimethylprop-2-ynyl)-N-heptyl-heptan-1-amine sont obtenus sous forme d'huile incolore, rdt 27%.

Le produit final est obtenu à partir de 0.51 g (1.82 mmol) de N-(1,1-diméthylprop-2-ynyl)-N-heptyl-heptan-1-amine avec un rendement de 29% sous forme de liquide (base libre) (0.187 g).

### Composé obtenu à la fin de l'étape e) (S-methyl 4-(diheptylamino)-4-methyl-pent-2-ynethioate)

- **¹³C NMR** (75 MHz, CDCl₃) δ 176.50 (C), 98.81 (C), 80.18 (C), 54.80 (C), 51.21 (CH₂), 31.94 (CH₂), 30.05 (CH₂), 29.29 (CH₂), 28.61 (CH₃), 27.45 (CH₂), 22.67 (CH₂), 14.13 (CH₃), 12.41 (CH₃).
- **ESI-LRMS** 354.1 [M+H]⁺.

## Revendications

1. Procédé de préparation de composés de formule (I) 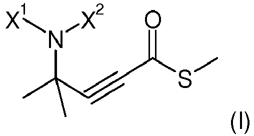 dans laquelle X¹ et X², identiques ou différents, sont choisis parmi les alkyles en C₁ à C₇, le phényle, le benzyle ou X¹ et X² forment avec l'atome d'azote qui les porte, un heterocycle, ,
le procédé comprenant les étapes suivante :
a) réaction d'un composé de formule (II) avec un acide inorganique ou un acide organique 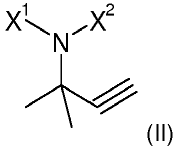
b) réaction du composé obtenu à l'étape a) avec une base présentant un pKa supérieur à 25;
c) réaction du composé obtenu à l'étape b) avec du CO₂ ;
d) réaction du composé obtenu à l'étape c) avec un chloroformiate d'alkyle, un réactif susceptible de former, avec le composé obtenu à l'étape c) un halogénure d'acide ou un réactif susceptible de former, avec le composé obtenu à l'étape c), un anhydride mixte ;
e) réaction du composé obtenu à l'étape d) avec un composé précurseur d'anion SMe⁻.

2. Procédé selon la revendication 1, dans lequel le composé de formule (II) est obtenu par une étape a1) de réaction du 3-chloro-3-méthylbut-1-yne avec X¹X²NH dans un milieu aqueux.

3. Procédé selon la revendication 2, dans lequel le composé obtenu à l'étape a1) est purifié par une ou plusieurs filtrations.

4. Procédé selon la revendication 2 ou 3, dans lequel le 3-chloro-3-méthylbut-1-yne est obtenu par une étape a0) de réaction du 2-méthylbut-3-yn-2-ol avec de l'acide chlorhydrique en présence d'un catalyseur au cuivre.

5. Procédé selon l'une quelconque des revendications 1 à 4, dans lequel l'acide est un acide inorganique choisi parmi l'acide chlorhydrique, l'acide phosphorique, l'acide nitrique, l'acide sulfurique.

6. Procédé selon l'une quelconque des revendications 1 à 5, dans lequel la base de l'étape b) est choisie parmi les bases à base de lithium ou de magnésium.

7. Procédé selon l'une quelconque des revendications 1 à 6, dans lequel l'étape d) est mise en œuvre avec :
- un chloroformiate d'alkyle présentant un alkyle en 1 à 6 carbone, pouvant comprendre au moins une double liaison ; ou
- un réactif susceptible de former avec le composé obtenu à l'étape c) un anhydride mixte choisi parmi les chlorures d'acide ; ou
- un réactif susceptible de former avec le composé obtenu à l'étape c) un halogénure d'acide choisi parmi SOCl₂, COCl₂, PCl₃, PCl₅, PBr₃ ou PPh₃Br₂.

8. Procédé selon l'une quelconque des revendications 1 à 7, pour lequel les composés précurseurs d'anion SMe⁻ sont choisis parmi les sels de formule XSMe dans laquelle X représente un métal alcalin ou alcalino-terreux.

9. Procédé de préparation d'un sel d'un composé de formule (I) tel que décrit à la revendication 1, comprenant les étapes de :
i) préparation du composé de formule (I) selon le procédé des revendications 1 à 8 ;
ii) réaction du composé obtenu à l'étape i) avec l'acide correspondant au sel souhaité.

10. Procédé selon l'une quelconque des revendications 1 à 8, dans lequel X¹ et X², identiques, représentent un méthyle.

11. Procédé selon la revendication 9, dans lequel ledit sel est le fumarate, dans lequel ledit composé de formule (I) possède X¹ et X², identiques, représentant un méthyle, et dans lequel l'acide mis en œuvre à l'étape ii) est l'acide fumarique.

12. Procédé selon la revendication 1, dans lequel X¹ et X² forment avec l'atome d'azote qui les porte, un hétérocycle pipéridine ou morpholine.

13. Procédé selon l'une quelconque des revendications 1 à 12, dans lequel le composé obtenu à l'étape a1) est purifié par 1 filtration ou en une succession de 2 à 10 filtrations.

14. Procédé selon l'une quelconque des revendications 1 à 13, dans lequel la base de l'étape b) est choisie parmi le butyllithium, ou l'hexyllithium.

15. Procédé selon l'une quelconque des revendications 1 à 14, pour lequel les composés précurseurs d'anion SMe⁻ sont choisis parmi Na, le methyl mercaptan, ou (SMe)₂.

## Patentansprüche

1. Verfahren zur Herstellung von Verbindungen der Formel (I) 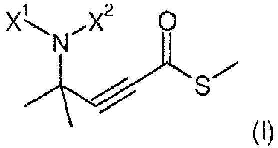 wobei X¹ und X², gleich oder verschieden, ausgewählt sind aus C₁-Alkyl bis C₇-Alkyl, Phenyl, Benzyl, oder X¹ und X² zusammen mit dem sie tragenden Stickstoffatom einen Heterozyklus bilden,
wobei das Verfahren die folgenden Schritte umfasst:
a) Umsetzen einer Verbindung der Formel (II) mit einer anorganischen Säure oder einer organischen Säure 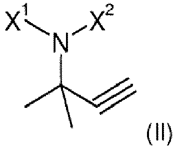
b) Umsetzen der in Schritt a) erhaltenen Verbindung mit einer Base mit einem pKa größer als 25;
c) Umsetzen der in Schritt b) erhaltenen Verbindung mit CO₂;
d) Umsetzen der in Schritt c) erhaltenen Verbindung mit einem Alkylchlorformiat und einem Reagenz, das anfällig dafür ist, mit der in Schritt c) erhaltenen Verbindung ein Säurehalogenid zu bilden oder einem Reagenz, das anfällig dafür ist, mit der in Schritt c) erhaltenen Verbindung ein gemischtes Anhydrid zu bilden;
e) Umsetzen der in Schritt d) erhaltenen Verbindung mit einer Vorläuferverbindung des Anions SMe⁻.

2. Verfahren nach Anspruch 1, bei dem die Verbindung der Formel (II) durch einen Schritt a1) der Umsetzung von 3-Chlor-3-methylbut-1-in mit X¹X²NH in einem ein wässrigen Medium erhalten wird.

3. Verfahren nach Anspruch 2, bei dem die in Schritt a1) erhaltene Verbindung durch eine oder mehrere Filtrationen gereinigt wird.

4. Verfahren nach Anspruch 2 oder 3, bei dem 3-Chlor-3-methylbut-1-in durch einen Schritt a0) der Umsetzung von 2-Methylbut-3-in-2-ol mit Salzsäure in Gegenwart eines Kupferkatalysators erhalten wird.

5. Verfahren nach einem der Ansprüche 1 bis 4, wobei die Säure eine anorganische Säure ausgewählt aus Salzsäure, Phosphorsäure, Salpetersäure, Schwefelsäure ist.

6. Verfahren nach einem der Ansprüche 1 bis 5, bei dem die Base von Schritt b) ausgewählt ist aus Lithium- oder Magnesium-basierten Basen.

7. Verfahren nach einem der Ansprüche 1 bis 6, wobei Schritt d) durchgeführt wird mit:
- einem Alkylchlorformiat, das ein Alkyl mit 1 bis 6 Kohlenstoffatomen aufweist und mindestens eine Doppelbindung enthalten kann; oder
- einem Reagenz, das anfällig ist, mit der in Schritt c) erhaltenen Verbindung ein gemischtes Anhydrid zu bilden, das ausgewählt ist aus Säurechloriden; oder
- einem Reagenz, das anfällig ist, mit der in Schritt c) erhaltenen Verbindung ein Säurehalogenid zu bilden, das ausgewählt ist aus SOCl₂, COCl₂, PCl₃, PCl₅, PBr₃ oder PPh₃Br₂.

8. Verfahren nach einem der Ansprüche 1 bis 7, für das die Vorläuferberbindungen des Anions SMe⁻ ausgewählt sind aus Salzen der Formel XSMe, wobei X ein Alkalimetall oder Erdalkalimetall darstellt.

9. Verfahren zur Herstellung eines Salzes einer Verbindung der wie in Anspruch 1 beschriebenen Formel (I), umfassend die Schritte:
i) Herstellen der Verbindung der Formel (I) gemäß dem Verfahren der Ansprüche 1 bis 8;
ii) Umsetzen der in Schritt (i) erhaltenen Verbindung mit der Säure, die dem gewünschten Salz entspricht.

10. Verfahren nach einem der Ansprüche 1 bis 8, wobei X¹ und X², identisch zueinander, ein Methyl darstellen.

11. Verfahren nach Anspruch 9, bei dem das Salz Fumarat ist, bei dem die Verbindung der Formel (I) X¹ und X² besitzt, die identisch sind, wobei sie ein Methyl darstellen, und bei dem die in Schritt ii) verwendete Säure Fumarsäure ist.

12. Verfahren nach Anspruch 1, bei dem X¹ und X² zusammen mit dem Stickstoffatom, das sie tragen, einen Piperidin- oder Morpholin-Heterozyklus bilden.

13. Verfahren nach einem der Ansprüche 1 bis 12, bei dem die in Schritt a1) erhaltene Verbindung durch 1 Filtration oder in einer Abfolge von 2 bis 10 Filtrationen gereinigt wird.

14. Verfahren nach einem der Ansprüche 1 bis 13, bei dem die Base von Schritt b) ausgewählt ist aus Butyllithium oder Hexyllithium.

15. Verfahren nach einem der Ansprüche 1 bis 14, bei dem die Vorläuferverbindungen des Anions SMe⁻ ausgewählt sind aus Na, Methylmercaptan oder (SMe)₂.

## Claims

1. Method for the preparation of compounds of formula (I) 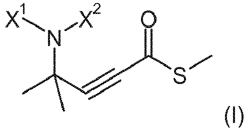 in which X¹ and X², which are identical or different, are chosen from C₁ to C₇ alkyls, phenyl, benzyl, or X¹ and X² form with the nitrogen atom, which carries them, a heterocycle, wherein the method comprises the following steps:
a) reaction of a compound of formula (II) with an inorganic acid or an organic acid 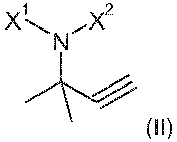
b) reaction of the compound obtained in step a) with a base having a pKa greater than 25;
c) reaction of the compound obtained in step b) with CO₂;
d) reaction of the compound obtained in step c) with an alkyl chloroformate, a reagent capable of forming, with the compound obtained in step c), an acid halide or a reagent capable of forming, with the compound obtained in step c), a mixed anhydride;
e) reaction of the compound obtained in step d) with an SMe⁻ anion precursor compound.

2. Method according to claim 1, wherein the compound of formula (II) is obtained by a step a1) of reaction of 3-chloro-3-methylbut-1-yne with X¹X²NH in an aqueous medium.

3. Method according to claim 2, wherein the compound obtained in step a1) is purified by one or more filtrations.

4. Method according to claim 2 or 3, wherein the 3-chloro-3-methylbut-1-yne is obtained by a step a0) of reaction of 2-methylbut-3-yn-2-ol with hydrochloric acid in the presence of a copper catalyst.

5. Method according to any one of the claims 1 to 4, wherein the acid is an inorganic acid selected from hydrochloric acid, phosphoric acid, nitric acid, sulfuric acid.

6. Method according to any one of the claims 1 to 5, wherein the base of step b) is chosen from bases based on lithium or magnesium.

7. Method according to any one of the claims 1 to 6, wherein step d) is implemented with:
- an alkyl chloroformate having a 1-6 carbon alkyl, which may comprise at least one double bond; or
- a reagent capable of forming with the compound obtained in stage c) a mixed anhydride chosen from acid chlorides; or
- a reagent capable of forming with the compound obtained in stage c) an acid halide chosen from SOCl₂, COCl₂, PCl₃, PCl₅, PBr₃ or PPh₃Br₂.

8. Method according to any one of the claims 1 to 7, wherein the SMe⁻ anion precursor compounds are selected from salts of formula XSMe, wherein X represents an alkali metal or alkaline earth metal.

9. Method for preparing a salt of a compound of formula (I) as described in claim 1, comprising the steps of:
i) preparation of the compound of formula (I) according to the method of claims 1 to 8;
ii) reaction of the compound obtained in step i) with the acid corresponding to the desired salt.

10. Method according to any one of the claims 1 to 8, wherein X¹ or X², which are identical, represent a methyl.

11. Method according to claim 9, wherein said salt is fumaric, wherein said compound of formula (I) has X¹ and X², identical, representing a methyl, and in which the acid used in step ii) is fumaric acid.

12. Method according to claim 1, wherein X¹ and X² form with the nitrogen atom which carries them, a heterocycle piperidine or morpholine

13. Method according to any of claims 1 to 12, wherein the compound obtained in step a1) is purified by one filtration or in a succession of 2 to 10 filtrations.

14. Method according to any of claims 1 to 13, wherein the base of step b) is chosen from butyllithium, or hexyllithium.

15. Method according to any of claims 1 to 14, wherein anion precursor compounds SMe⁻ are selected from Na, the methyl mercaptan, or (SMe)₂.
